# EUROPEAN PATENT SPECIFICATION

(11) **EP 2 981 316 B1**
(45) Date of publication and mention of the grant of the patent: **08.06.2022**
(21) Application number: 14778422.7
(22) Date of filing: 19.03.2014
(51) Int. Cl.: A61M 5/315

(54) **SYRINGE AND AN ACCESSORY THEREFOR**
SPRITZE UND ZUBEHÖR DAFÜR
SERINGUE ET ACCESSOIRE POUR CELLE-CI

(30) Priority: 03.04.2013 ZA 201302385
(43) Date of publication of application: 10.02.2016
(73) Proprietor: Armstrong, Sean Terrence, Bedfordview 2007 (ZA)
(72) Inventor: Armstrong, Sean Terrence, Bedfordview 2007 (ZA)
(74) Representative: Fry, David John
(86) International application number: PCT/ZA2014/000011
(87) International publication number: WO 2014/165868

(56) References cited:
- WO-A1-2009/095735
- WO-A1-2009/095735
- WO-A2-2008/057976
- US-A1- 2011 009 829
- US-A1- 2011 009 829
- US-B1- 6 579 269

## Description

### BACKGROUND

The present invention relates to an accessory for a syringe. More specifically, the present invention relates to an accessory that, when attached to a syringe, generates audible clicks as the plunger rod moves along the barrel of the syringe. Even more specifically, the present invention relates to an accessory that can be secured to the finger flanges or barrel of a syringe and receive a syringe plunger while the plunger rod is located within the barrel of the syringe. The invention also relates to a syringe including the accessory. Syringes with mechanisms for regulating or monitoring movement of a plunger along a barrel are known. For instance:
US3,934,586 to Easton describes a syringe with tabs on the plunger rod that engage with wedges extending radially inwards from the barrel;
DE807,113 describes a syringe with tabs extending from the plunger rod and a regulator attachable to the outer perimeter of the barrel to regulate relative movement of the plunger rod and barrel; and
US4,642,102 to Ohmori describes a stopper secured to and incrementally movable along the plunger rod, which stopper limits relative movement of the plunger rod and barrel when the stopper comes into contact with the barrel.

A syringe finger flange cover through which a syringe plunger rod is received and that regulates movement of the plunger rod therethrough is also known. For instance, US5,318,544 to Drypen et al describes such a cover defining a central bore for receiving the plunger rod. However, the cover disclosed in that patent requires that the plunger rod be removed from the syringe barrel, that the cover be secured to the syringe finger flanges, and that the plunger rod be re-inserted into the syringe barrel via the bore defined by the cover. A drawback of this arrangement is that removal of the plunger rod from the syringe barrel could adversely impacts sterility of the syringe. WO 2008/057976 A2 describes a syringe comprising the combination of features of the preamble of claim 1. WO 2009/095735 A1 describes a syringe with an accessory having a body that can be mounted to the syringe without requiring removal of the plunger rod from the syringe barrel.

It is an object of the present invention to provide an alternative accessory for a syringe that includes a body that secures to the finger flanges or barrel of a syringe without requiring removal of the plunger rod from the syringe barrel and that creates audible clicks as the plunger rod moves along the syringe barrel.

### SUMMARY OF THE INVENTION

The invention comprises a syringe including an accessory with a body according to claim 1. Preferred features of the invention are defined in the dependent claims.

The body includes a first finger extending from the body into the primary bay, the first finger being the portion of the body that engages the array of notches and/or protuberances on the plunger rod.

In use, the body is secured to the finger flanges of a syringe.

Further, the body includes a substantially planar portion that, in use, locates over a planar surface of finger flanges on a syringe and defines the primary bay.

Generally, the securing means includes a lip that extends substantially orthogonally from at least a portion of the peripheral end of the substantially planar portion.

Preferably, the securing means comprises at least one tab that extends inwards from the free end of the lip.

Typically, the at least one tab comprises an operatively lower planar segment that defines a secondary bay that corresponds with the outer diameter of a syringe barrel.

Generally, the lip spaces the substantially planar portion and the tab a distance corresponding to the width of a syringe finger flange.

Preferably, the lip spaces the substantially planar portion and the tab by between 1.5mm and 3mm.

Typically, the securing means permits the body to be slidable located over the finger flanges of a syringe.

According to the invention, the accessory is used with a plunger rod that in section orthogonal to the longitudinal axis of the plunger rod, has a shape wherein four equally spaced arms radiate radially from the longitudinal axis of the plunger rod.

Further, the primary bay includes a linear channel that is sized and shaped to correspond with a first plunger rod arm so as, in use, to receive the first plunger rod arm therein.

The finger is located at or near the closed end of the linear channel. Generally, in use, the nipple bears against a second plunger rod arm when the first plunger rod arm is received within the linear channel.

Further, in use, a second nipple bears against a third plunger rod arm when the first plunger rod arm is received within the linear channel.

According to the invention, the primary bay converges from the perimeter of the substantially planar portion towards the first and second nipples, in use to guide a plunger rod along the primary bay.

According to the present invention, the syringe includes:
a barrel;
finger flanges extending radially from the barrel;
a plunger rod including an array of notches and/or protuberances along at least a portion of its length; and
the accessory with the body located over the finger flanges and the plunger rod received and captured within the bay.

The plunger rod in section orthogonal to the longitudinal axis of the plunger rod, has a shape wherein four equally spaced arms radiate radially from the longitudinal axis of the plunger rod.

Preferably, the free end of at least one arm is chamfered to facilitate movement of that arm past the nipple.

### BRIEF DESCRIPTION OF THE DRAWINGS

The invention will now be described in more detail, by way of example only, with reference to the accompanying drawings in which:
- **Figure 1**: is an upper perspective view of an accessory for a syringe according to a first aspect of the invention;
- **Figure 2**: is a lower perspective view of the accessory in Figure 1;
- **Figure 3**: is a plan view of the accessory in Figure 1;
- **Figure 4**: is a perspective view of a syringe according to a second aspect of the invention;
- **Figure 5**: is a perspective view of the syringe in Figure 4 with the accessory positioned prior to being slidably located over the finger flanges of the syringe; and
- **Figure 6**: is a perspective view of an alternative embodiment of the accessory according to the present invention.

In this specification, "bay" means an open-ended slot / an aperture defined by a body that extends to the perimeter of the body.

### DESCRIPTIONS OF THE INVENTION

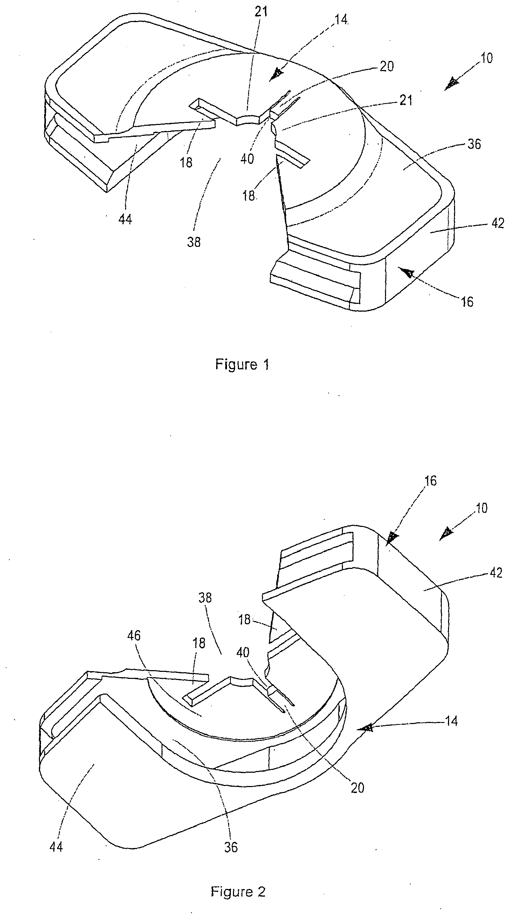

With reference to Figures 1 to 5, an accessory 10 for a syringe 12, according to a first aspect of the invention includes a body 14, securing means 16, nipples 18 and a finger 20.

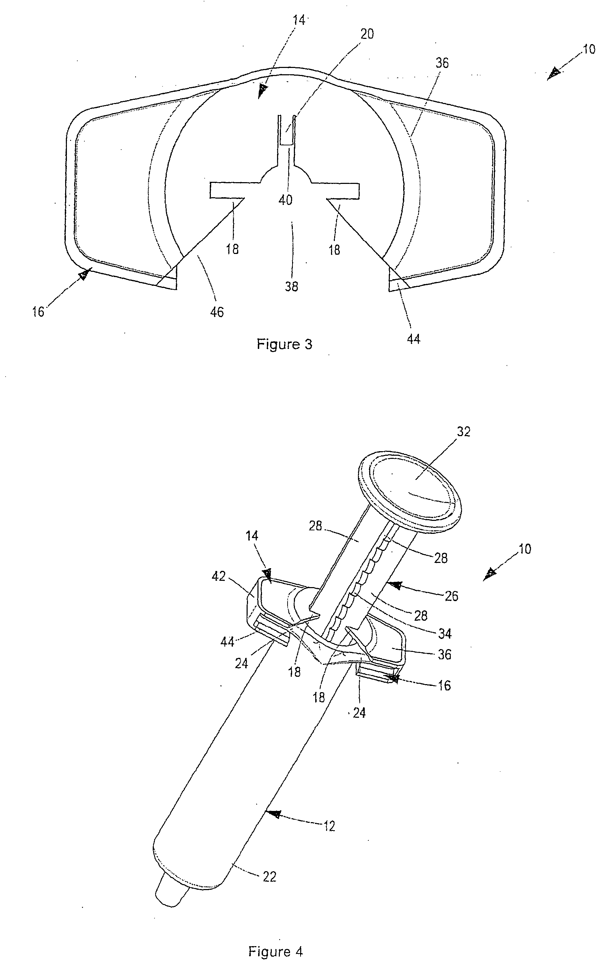

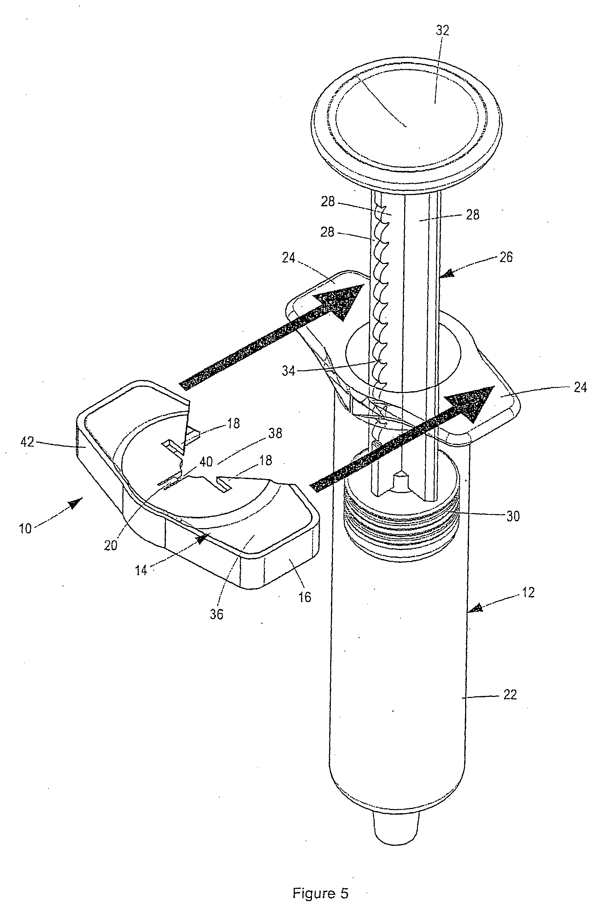

With reference to Figures 4 and 5, the syringe 12 is a standard syringe comprising a barrel 22 and a pair of finger flanges 24 extending diametrically from the barrel 22.

The syringe 12 includes a plunger rod 26 that, in section orthogonal to its longitudinal axis, has a shape wherein four equally spaced arms 28 of the same length radiate from the longitudinal axis (i.e. in the shape of an "X"). A plunger 30 is secured to the plunger rod 26 at one end and the other end of the plunger rod 26 terminates in an enlarged head 32 in the shape of a disc.

The free end of one arm 28 of the plunger rod 26 includes an array of notches and/or protuberances 34 extending along a portion of the length of the plunger rod 26. It will be appreciated that although the plunger rod 26 has been shown with an array of notches and/or protuberances 34 along one arm 28, it will be appreciated that an array of notches and/or protuberances 34 could alternatively be located on two or three arms 28.

Furthermore, it will be appreciated that the notches and/or protuberances 34 could be located at the point of intersection of the plunger rod arms 28. In such an arrangement, formation(s) 21 could constitute the finger(s) 20 that engage such notches and/or protuberances 34 on the plunger rod 26.

The free (i.e. radial) end of at least one arm 28 is chamfered to facilitate movement of that arm 28 past the nipple 18.

Turning back to the accessory 10 and Figures 1 to 3, the body 14 is sized and shaped, in use, to locate over finger flanges 24 of the syringe 12. Although not shown in the Figures, it will be appreciated that the accessory 10 could alternatively be secured to the barrel 22 of the syringe 12.

The body 14 includes a substantially planar portion 36 that is, in use, in facial contact with the operative upper planar portions of the finger flanges 24. The substantially planar portion 36 defines a primary bay 38 that extends from the perimeter of the substantially planar portion 36 into the substantially planar portion 36, the primary bay 38 defining an opening for receiving and releasably capturing the plunger rod 26 therein.

The primary bay 38 defined by the substantially planar portion 36 is sized and shaped such that, in use, when the body 14 is secured to the syringe finger flanges 24 and the plunger rod 26 is received and releasably captured within the primary bay 38, the longitudinal axis of the plunger rod 26 is aligned with the longitudinal axis of the syringe barrel 22.

The primary bay 38 converges from its open end towards the pair of nipples 18, which nipples 18 form a constriction in the primary bay 38. The width of the primary bay 38 at the nipples 18 is slightly smaller than the width of the plunger rod 26. As such, either the body 14 or the nipples 18 must resiliently flex / distort to permit the plunger rod 26 to pass the nipples 18 and continue along the primary bay 38. A linear channel 40 extends from the end of the primary bay 38 into the substantially planar portion 36. The linear channel 40 is sized and shaped to receive a plunger rod arm 28 therein. The finger 20 is located near the closed end of the channel 40.

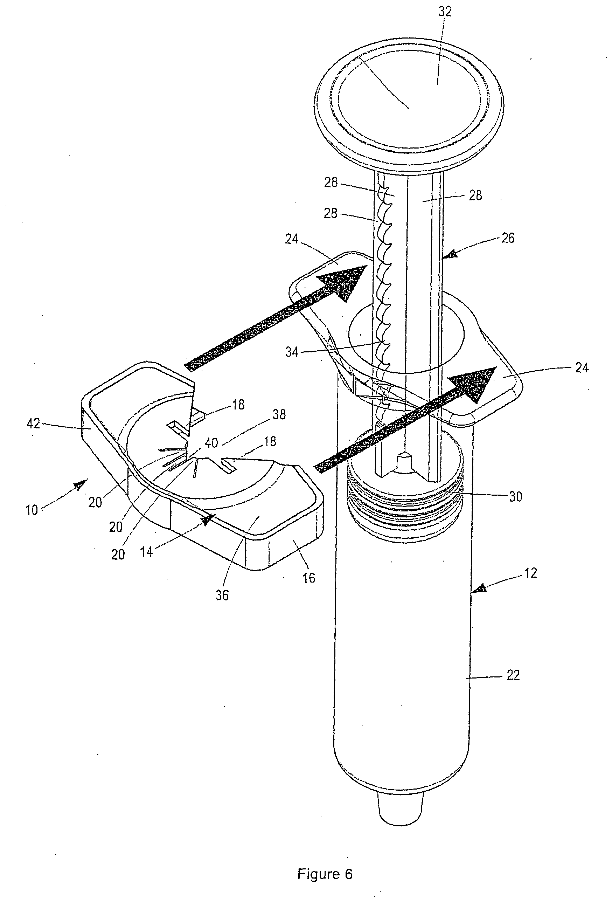

An alternative embodiment of the accessory 10 is showin in Figure 6. This embodiment includes a series of three fingers 20 that engage the notches and/or prituberances on the plunger rod 26 arm 28.

It will be appreciated that if a second or third plunger rod arm 28 includes notches and/or protuberances 34, corresponding second or third fingers 20 could extend from the body 14 in the vicinity of such arms 28.

When a plunger rod 26 is received within the primary bay 38, the plunger rod 26 is guided by the converging primary bay 38 towards the nipples 18. The plunger rod 26 is then forced past the nipples 18 and one of the plunger rod arms 28 (with notches and protuberances 34) is received into the linear channel 40 with the finger 20 engaging the notches and protuberances 34. With one plunger rod arm 28 received within the linear channel 40, two other plunger rod arms 28 bear against the nipples 38, acting to releasably capture the plunger rod 26 within the primary bay 38. So captured within the primary bay 38, the plunger rod 26 may move axially along the syringe barrel 22 with finger 20 running along the notches and/or protuberances 34 on the plunger rod arm 26 to generate audible clicks.

Preferably, the fingers 20 are sufficiently thin and flexible to generate audible clicks without materially resisting movement of the plunger rod 26 relative to the accessory 10. The audible clicks assist the doctor to administer the correct dosage to the patient, and satisfy the patient that the dosage requested / prescribed has been administered.

It will be appreciated that the accessory 10 could dispense with the finger 20. In such an arrangement (not shown in the Figures), the notches and/or protuberances 34 on the plunger rod arm 26 could interact directly with a portion of the body 14 to generate audible clicks.

A lip 42 extends orthogonally from the peripheral end of the planar portion 36, in use, covering the sides of the finger flanges 24. Tabs 44 extend inwards from or near the free end of the lip 42. The Figures show the tabs 44 in the form of an operatively lower planar segment extending between the curved lip 42. The planar segment 44 defines a circular secondary bay 46 that corresponds with the radial outer peripheral surface of the syringe barrel 22 (i.e. has a diameter corresponding to the outer diameter of the barrel 22). As such, when the accessory 10 is located over the syringe finger flanges 24, the substantially planar portion overlies the operatively upper surface of the finger flanges 24; the lip 42 overlies the sides of the finger flanges 24, and the planar segment 44 overlies the operatively lower surface of the finger flanges 24 - the lip 42 spacing the planar portion 36 from the planar segment 44 by the width of the finger flanges 24 (i.e. between 1.5mm and 3mm).

Referring to Figure 5, the accessory 10 is located over the finger flanges 24 by aligning the secondary bay 46 with the syringe barrel 22 and the primary bay 38 with the plunger rod 26, and sliding the accessory 10 sideways over the finger flanges 24.

The lip 42 and tabs 44 make up the securing means 16 that secure the body 14 to the finger flanges 24. However, it will be appreciated that the securing means 16 could comprise an ultrasonic weld that welds the body 14 to the finger flanges 24 or barrel 22.

Referring to Figures 4 and 5, according to a second aspect of the invention, there is provided a syringe 12 including: a barrel 22; finger flanges 24 extending radially from the barrel 22; a plunger rod 26 including an array of notches and/or protuberances 34 along at least a portion of its length; and an accessory 10 according to the first aspect of the invention. The accessory 10 locates over the finger flanges 24 and the plunger rod 26 is received and captured within the primary bay 38 defined by the planar portion 36 of the accessory 10 body 14.

It will be appreciated that fitment of the accessory 10 to the finger flanges 24 and syringe plunger 26 does not require removal of the plunger rod 26 from the syringe barrel 22, thereby maintaining sterility of the syringe 12. Furthermore, by capturing the plunger rod 26 within the primary bay 38, the accessory 10 also limits lateral movement of the plunger rod 26 as it is depressed axially into the syringe barrel 22.

## Claims

1. A syringe (12) including:
a barrel (22);
finger flanges (24) extending radially from the barrel which define a planar surface;
a plunger rod (26) which, in section orthogonal to the longitudinal axis of the plunger rod, has a shape defined by four equally spaced arms radiate radially from the longitudinal axis of the plunger rod, a first arm of which includes an array of notches (34) and/or protuberances along at least a portion of its length;
wherein the syringe includes an accessory (10) including a body (14) sized and shaped in use to locate over the finger flanges and securing means, to secure the body to the finger flanges, wherein the body includes a planar portion (36) that locates over the planar surface of finger flanges and which defines a primary bay (38) in which the plunger rod is received in use, **characterized by** Z the primary bay having a linear channel (40) that is sized and shaped to correspond with the first plunger rod arm to receive the first plunger rod arm therein;
wherein the body further includes a first finger (20) integrally formed with the body and extending from the body into the primary bay, at or near the closed end of the linear channel the first finger engaging the array of notches and/or protuberance on the first arm of the plunger rod to create audible clicks when the plunger rod moves along the syringe barrel;
and wherein the primary bay further includes a first nipple (18) integrally formed with the body and extending from the body into the primary bay, the first nipple forming a constriction along the primary bay and bearing against a second plunger rod arm (28) during assembly of the syringe;
and wherein the primary bay further includes a second nipple (18) integrally formed with the body and extending from the body into the primary bay, and forming a constriction along the primary bay and bearing against a third plunger rod arm (28) during assembly of the syringe;
and wherein the primary bay is sized and shaped such that the width of the primary bay at the first and second nipples is smaller than the width of the plunger rod thereby causing the body and/or the first and second nipples to resiliently flex or distort in order to permit the plunger rod to pass the first and second nipples and continue through the primary bay;
and wherein the plunger rod is received and captured within the primary bay with the longitudinal axis of the plunger rod aligned with the longitudinal axis of the syringe barrel;
and wherein the primary bay converges from the perimeter of the planar portion towards the first and second nipples to facilitate guiding of the plunger rod along the primary bay during assembly of the syringe.

2. A syringe according to claim 1, wherein the securing means includes a lip (42) that extends substantially orthogonally from at least a portion of the peripheral end of the planar portion; and comprises at least one tab that extends inwards from the free end of the lip.

3. A syringe according to claim 2, wherein the at least one tab (44) comprises an operatively lower planar segment that defines a secondary bay that corresponds with the outer diameter of a syringe barrel.

4. A syringe according to claim 3, wherein the lip spaces the planar portion and the tab by between 1 .5 mm and 3 mm.

5. A syringe according to claim 4, wherein the securing means permits the body to be slidably located over the finger flanges of a syringe.

6. A syringe according to claim 5, wherein the free ends of the second and third plunger rod arms are chamfered to facilitate movement of the second and third plunger rod arms past the first and second nipples, respectively.

## Patentansprüche

1. Spritze (12), die Folgendes einschließt:
einen Zylinder (22);
Fingerflansche (24), die sich radial von dem Zylinder erstrecken und eine ebene Oberfläche definieren;
eine Kolbenstange (26), die, im Querschnitt orthogonal zur Längsachse der Kolbenstange, eine Form aufweist, die durch vier gleich beabstandete Arme definiert ist, die radial von der Längsachse der Kolbenstange ausstrahlen, wobei ein erster Arm davon eine Anordnung von Kerben (34) und/oder Vorsprüngen entlang mindestens eines Abschnitts seiner Länge einschließt;
wobei die Spritze ein Zubehörteil (10) einschließt, das einen Körper (14), der bei der Verwendung so bemessen und geformt ist, dass er über den Fingerflanschen positioniert ist, und ein Sicherungsmittel zum Sichern des Körpers an den Fingerflanschen einschließt, wobei der Körper einen ebenen Abschnitt (36) einschließt, der über der ebenen Oberfläche der Fingerflansche positioniert ist und eine Primärbucht (38) definiert, in welcher die Kolbenstange bei der Verwendung aufgenommen ist, **dadurch gekennzeichnet, dass**
die Primärbucht einen linearen Kanal (40) aufweist, der so bemessen und geformt ist, dass er zu dem ersten Kolbenstangenarm passt, um den ersten Kolbenstangenarm darin aufzunehmen;
wobei der Körper ferner einen ersten Finger (20), der einstückig mit dem Körper ausgebildet ist und sich von dem Körper in die Primärbucht erstreckt, an oder nahe dem geschlossenen Ende des linearen Kanals einschließt, wobei der erste Finger in die Anordnung von Kerben und/oder Vorsprüngen an dem ersten Arm der Kolbenstange eingreift, um hörbare Klicks zu erzeugen, wenn sich die Kolbenstange entlang des Spritzenzylinders bewegt;
und wobei die Primärbucht ferner einen ersten Nippel (18) einschließt, der einstückig mit dem Körper ausgebildet ist und sich von dem Körper in die Primärbucht erstreckt, wobei der erste Nippel eine Verengung entlang der Primärbucht ausbildet und während des Zusammenbaus der Spritze gegen einen zweiten Kolbenstangenarm (28) anliegt;
und wobei die Primärbucht ferner einen zweiten Nippel (18) einschließt, der einstückig mit dem Körper ausgebildet ist und sich von dem Körper in die Primärbucht erstreckt und eine Verengung entlang der Primärbucht ausbildet und während des Zusammenbaus der Spritze gegen einen dritten Kolbenstangenarm (28) anliegt;
und wobei die Primärbucht so bemessen und geformt ist, dass die Breite der Primärbucht am ersten und zweiten Nippel kleiner ist als die Breite der Kolbenstange, wodurch bewirkt wird, dass sich der Körper und/oder der erste und zweite Nippel elastisch biegen oder verformen, um es der Kolbenstange zu ermöglichen, den ersten und den zweiten Nippel zu passieren und sich durch die Primärbucht fortzusetzen;
und wobei die Kolbenstange innerhalb der Primärbucht aufgenommen und festgehalten wird, wobei die Längsachse der Kolbenstange an der Längsachse des Spritzenzylinders ausgerichtet ist;
und wobei die Primärbucht von dem Umfang des ebenen Abschnitts hin zu dem ersten und zweiten Nippel konvergiert, um ein Führen der Kolbenstange entlang der Primärbucht während des Zusammenbaus der Spritze zu erleichtern.

2. Spritze nach Anspruch 1, wobei das Sicherungsmittel eine Lippe (42) einschließt, die sich im Wesentlichen orthogonal von mindestens einem Abschnitt des peripheren Endes des ebenen Abschnitts erstreckt; und mindestens eine Lasche umfasst, die sich von dem freien Ende der Lippe nach innen erstreckt.

3. Spritze nach Anspruch 2, wobei die mindestens eine Lasche (44) ein operativ unteres planares Segment umfasst, das eine Sekundärbucht definiert, die zu dem Außendurchmesser eines Spritzenzylinders passt.

4. Spritze nach Anspruch 3, wobei die Lippe den ebenen Abschnitt und die Lasche um zwischen 1,5 mm und 3 mm beabstandet.

5. Spritze nach Anspruch 4, wobei das Sicherungsmittel es ermöglicht, dass der Körper verschiebbar über den Fingerflanschen einer Spritze positioniert ist.

6. Spritze nach Anspruch 5, wobei die freien Enden des zweiten und dritten Kolbenstangenarms angeschrägt sind, um eine Bewegung des zweiten und dritten Kolbenstangenarms an dem ersten bzw. zweiten Nippel vorbei zu erleichtern.

## Revendications

1. Seringue (12) comprenant :
un cylindre (22) ;
des rebords de doigt (24) s'étendant radialement depuis le cylindre qui définissent une surface plane ;
une tige de piston (26) qui, en coupe orthogonale à l'axe longitudinal de la tige de piston, a une forme définie par quatre bras équidistants rayonnant radialement à partir de l'axe longitudinal de la tige de piston, dont un premier bras comprend un ensemble d'encoches (34) et/ou des protubérances le long d'au moins une partie de sa longueur ;
ladite seringue comprenant un accessoire (10) qui comprend un corps (14) dimensionné et façonné lors de l'utilisation pour se placer sur les rebords de doigt et le moyen de fixation, pour fixer le corps aux rebords de doigt, ledit corps comprenant une partie plane (36) qui se trouve sur la surface plane des rebords de doigt et qui définit une baie primaire (38) dans laquelle la tige de piston est reçue lors de l'utilisation,
**caractérisé en ce que** la baie primaire comporte un canal linéaire (40) qui est dimensionné et façonné pour correspondre au premier bras de tige de piston pour recevoir le premier bras de tige de piston en son sein ;
ledit corps comprenant en outre un premier doigt (20) formé d'un seul tenant avec le corps et s'étendant du corps dans la baie primaire, au niveau ou à proximité de l'extrémité fermée du canal linéaire, le premier doigt engageant l'ensemble d'encoches et/ou une protubérance sur le premier bras de la tige du piston pour créer des clics audibles lorsque la tige du piston se déplace le long du cylindre de la seringue ;
et ladite baie primaire comprenant en outre un premier mamelon (18) formé d'un seul tenant avec le corps et s'étendant depuis le corps dans la baie primaire, le premier mamelon formant une constriction le long de la baie primaire et reposant contre un deuxième bras de tige de piston (28) lors du montage de la seringue ;
et ladite baie primaire comprenant en outre un second mamelon (18) formé d'un seul tenant avec le corps et s'étendant depuis le corps dans la baie primaire, et formant une constriction le long de la baie primaire et reposant contre un troisième bras de tige de piston (28) lors du montage de la seringue ;
et ladite baie primaire étant dimensionnée et façonnée de sorte que la largeur de la baie primaire au niveau des premier et second mamelons soit plus petite que la largeur de la tige de piston, ce qui provoque la flexion ou la déformation élastique du corps et/ou des premier et second mamelons afin de permettre à la tige de piston de passer les premier et second mamelons et de continuer à travers la baie primaire ;
et ladite tige de piston étant reçue et capturée à l'intérieur de la baie primaire, l'axe longitudinal de la tige de piston étant aligné avec l'axe longitudinal du corps de seringue ;
et ladite baie primaire convergeant depuis la périphérie de la partie plane vers les premier et second mamelons pour faciliter le guidage de la tige de piston le long de la baie primaire lors du montage de la seringue.

2. Seringue selon la revendication 1, ledit moyen de fixation comprenant une lèvre (42) qui s'étend sensiblement orthogonalement depuis au moins une partie de l'extrémité périphérique de la partie plane ; et comprenant au moins une languette qui s'étend vers l'intérieur depuis l'extrémité libre de la lèvre.

3. Seringue selon la revendication 2, ladite au moins une languette (44) comprenant un segment plan inférieur fonctionnel qui définit une baie secondaire qui correspond au diamètre externe d'un cylindre de seringue.

4. Seringue selon la revendication 3, ladite lèvre espaçant la partie plane et la languette d'une distance comprise entre 1,5 mm et 3 mm.

5. Seringue selon la revendication 4, ledit moyen de fixation permettant au corps d'être placé de manière coulissante sur les rebords des doigts d'une seringue.

6. Seringue selon la revendication 5, lesdites extrémités libres des deuxième et troisième bras de tige de piston étant chanfreinées pour faciliter le déplacement des deuxième et troisième bras de tige de piston devant les premier et seconds mamelons, respectivement.
